# EUROPEAN PATENT SPECIFICATION

(11) **EP 1 440 965 B1**
(45) Date of publication and mention of the grant of the patent: **25.02.2009**
(21) Application number: 04075604.1
(22) Date of filing: 28.08.1998
(51) Int. Cl.: C07C 311/06, C07C 317/44, A61K 31/18, A61K 31/22

(54) **Antimicrobial compounds**
Antimikrobielle Verbindungen
Composés antimicrobiens

(30) Priority: 29.08.1997 US 56272 P
(43) Date of publication of application: 28.07.2004
(62) Divisional of application: 98942276.1
(73) Proprietor: JOHNS HOPKINS UNIVERSITY, Baltimore, MD 21205 (US)
(72) Inventor: Townsend, Craig, Baltimore, Maryland 21212 (US); Dick, James, Upperco, Maryland 21155 (US); Pasternack, Gary, Baltimore 21210 (US); Kuhajda, Francis, Lutherville, Maryland 21093 (US); Parrish, Nicole, Ellicott City, Maryland 21042 (US)
(74) Representative: Jones, Nicholas Andrew

(56) References cited:
- WO-A-95/19706
- DE-A- 2 630 947
- DATABASE CHEMLIST AMERICAN CHEMICAL SOCIETY; STN, accession no. 32917 XP002096636 & OFFICIAL GAZETTE OF THE EUROPEAN COMMUNITIES, vol. ANNEX, 15 June 1990 (1990-06-15),

## Description

### Field of the Invention

This invention relates to the synthesis and *in vivo* application of compounds which have antibiotic activity against microbes that synthesize mycolic acid, including *Mycobacterium* sp particularly drug resistant *Mycobacterium* strains, and to the use of these compounds to treat any susceptible pathogenic microorganism or parasite.

### Review of Related Art

The emergence of multiply drug resistant (MDR) strains of *Mycobacterium tuberculosis* and other atypical mycobacteria which infect immunocompromised patients (e.g., AIDS patients) highlights the need for continued antibiotic development.

*Mycobacterium* sp. synthesize a multitude of complex lipids and glycolipids unique to this genus, making these biochemical pathways attractive targets for drug therapy (Bloch, K., "Control mechanisms for fatty acid synthesis in Mycobacterium smegmatis," Adv. Enzymol. 45:1-84, 1977; Brennan, P.J., and Nikaido, H., "The envelope of mycobacteria," Ann. Rev. Biochem. 64:29-63, 1995). The β-ketoacyl synthase (KS) of particulate Type II fatty acid synthases or the corresponding domain of the polyfunctional Type I fatty acid synthases catalyzes the critical two-carbon homologation during buildup of the growing fatty acid chain. This process typically gives acids of length C₁₆ to C₁₈. In chain elongation of normal fatty acids, carried out for example by mycobacteria, CoA and/or acyl-carrier protein (ACP) thioesters of these acids are further reacted with malonyl-CoA to greatly extend their length to 60-90 carbons. These high molecular weight acids are known collectively as mycolic acids.

Mycolic acids are a group of complex, long, branched chain fatty acids that are vital for the growth and survival of mycobacteria. Mycolic acids comprise the single largest component of the mycobacterial cell envelope. Little is known about the nature of the biosynthetic enzymes involved, but evidence suggests some similarity to conventional fatty acid synthases (Bloch, 1977; Brennan, et al., 1995). These unusually long lipid molecules form a waxy coat of limited permeability.

The presence in mycobacteria of particular modified fatty acids having complex and well-organized structures presents a potentially attractive target for drug design (Young, D.B., and Duncan, K., "Prospects for new interventions in the treatment and prevention of mycobacterial disease," Ann Rev. Microbiol. 49:641-673, 1995). It has been suggested that isoniazid inhibits mycolic acid synthesis as its potential mechanism of action (Takayama, K., Wang, L., and David, H.L., "Effect of isoniazid on the in vivo mycolic acid synthesis, cell growth, and viability of Mycobacterium tuberculosis," Antimicrob. Agents Chemother., 2:29-35,1972; Takayama, K., Schnoes, H.K., Armstrong, E.I., and Booyle, R.W., "Site of inhibitory action of isoniazid in the synthesis of mycolic acids in Mycobacterium tuberculosis," J. Lipid Res., 16:308-317, 1975; Quemard A., Dessen A., Sugantino M., Jacobs W.R., Sacchettini J.C., Blanchard J.S. "Binding of catalase peroxide-activated isoniazid to wild-type and mutant Mycobacterium tuberculosis enoyl-ACP reductases," J. Am. Chem. Soc., 118:1561-1562, 1996; Baldock C., Rafferty J.B., Sedenikova S.E., Baker P.J., Stuitje A.R., Slabas A.R., Hawkes T.R., Rice D.W. "A mechanism of drug action revealed by structural studies of enoyl reductase," Science, 274:2107-2110, 1996; Quemard A., Sacchettini J.C., Dessen A., Vilcheze C., Bittman R., Jacobs W.R., Blanchard J.S., "Enzymatic characterization of the target for isoniazid in Mycobacterium tuberculosis, Biochemistry, 34:8235-8241, 1993; Msluli, K., D.R. Sherman, M.J. Hickey, B.N. Kreiswirth, S. Morris, C.K. Stover, and C.E. Barry, III, "Biochemical and genetic data suggest that InhA is not the primary target for activated isoniazid in Mycobacterium tuberculosis," J. Infect. Dis., 174:1085-1090, 1996; Dessen A., A. Quemard, J.S. Blanchard, W.R. Jacobs, and J.C. Saccettini, "Crystal structure and function of the isoniazid target of Mycobacterium tuberculosis," Science, 267:1638-1641, 1995; Banerjee, A., E. Dubnau, A. Quemard, V. Balasubramanian, K.S. Um, T. Wilson, D. Collins, G. deLisle, W.R. Jacobs, Jr., "InhA, a gene encoding a target for isoniazid and ethionamide in Mycobacterium tuberculosis." Science, 263:227-230, 1994). This finding might be expected to stimulate a search for novel compounds that act upon the lipid synthetic pathways of mycobacteria as a fresh approach for antibiotic development. Surprisingly, however, lipid biosynthesis has not been exploited for drug development in these organisms. No drugs which specifically inhibit mycobacterial lipid synthesis have been developed other than isoniazid, and there remains a need for new drugs to treat the growing problem of multi-drug resistant mycobacteria.

In WO95/19706, the ability of cerulenin ((2*S*,3*R*)-2,3-epoxy-4-oxo-7, 10-dodecadienoylamide) to inhibit growth of *M. tuberculosis* isolates is demonstrated in *vitro.* The effects of cerulenin are said to be due to an inhibitory action on Type I/Type II fatty acid synthesis.

### SUMMARY OF THE INVENTION

This invention is directed to novel compounds having antimicrobial activity, particularly antimicrobial effectiveness against multi-drug resistant mycobacteria.

This invention is also directed to a method for treating mycobacterial infection by drug resistant strains through use of independent therapeutic targets.

These and other objects of the invention are achieved by one or more of the following embodiments. In one embodiment, this invention provides a compound having a formula:

R-SOₙ-Z-CO-Y

wherein:
R is an alkyl group having 6-20 carbon atoms or an alkyl group having 6-20 carbon atoms interrupted by an aromatic ring;
Z is a radical selected from the group consisting of -CH₂-, two of these radicals coupled together, and -CH=CH-;
Y is selected from -NH₂ and O-CH₂-C₆H₅; and
n is 1 or 2; and wherein the compound is not CH₃(CH₂)₁₁SO₂CH₂CONH₂. In particularly preferred embodiments, R is a branched alkyl group. Preferably, R is an alkyl group having 8, 10 or 12 carbon atoms.

In another embodiment, this invention provides a use of a compound having the formula:

R-SOₙ-Z-CO-Y

wherein:
R is selected from the group consisting of unsaturated hydrocarbon groups having 6-20 carbon atoms and alkyl groups having 6-20 carbon atoms interrupted by an aromatic ring;
Z is a radical selected from the group consisting of -CH₂-, -CH₂CH₂-, -NH-NH-, -O-, -NH-,-O-NH-, -CH₂-NH-, -CH₂O-, -NH-O-, -NH-CH₂-, -O-CH₂-, and -CH=CH-;
Y is selected from the group consisting of -NH₂, -O-CH₂-C₆H₅ and -O-CH₃; and
n is 1 or 2;
in the preparation of a medicament for inhibiting the growth of a microbial cell wherein the cell synthesizes α-substituted β-hydroxy fatty acids. In particular, cells inhibited are those which synthesize α-substituted, β-hydroxy fatty acids selected from the group consisting of corynemycolic acid, nocardic acid, and mycolic acid. Preferably, the medicament is used to inhibit growth of microbial cells selected from the group consisting of corynebacteria, nocardiae, rhodococcus, and mycobacteria. More preferably, the medicament is used to inhibit growth of mycobacterial cells, such as *Mycobacterium tuberculosis,* drug-resistant *M. tuberculosis, M. avium intracellulare, M. leprae,* or *M. paratuberculosis.*

Other preferred features of the present invention are detailed in the appended claims.

The present inventors have synthesized and tested a number of sulfones and sulfoxides having structures based upon the reaction intermediates of the β-ketoacyl synthase reaction of fatty acid synthase. A number of these compounds have demonstrated *in vitro* activity against virulent *M. tuberculosis* (see Table 1). The desirable characteristics found among the compounds tested included: potency, *in vivo* activity, reproducibility of MIC data, ease of synthesis, and chemical stability. Use of the compounds of this invention in drug therapy against multiply drug resistant tuberculosis will provide a means to treat both patients presently suffering from active disease, and the millions of potential patients who harbor quiescent disease which may become active as a result of immunosuppression or other systemic disease.

### BRIEF DESCRIPTION OF THE DRAWINGS

Figure 1 shows a schematic representation of the β-ketoacyl synthase reaction.
Figure 2 shows two-dimensional thin layer chromatography of mycolic acids extracted from *M*. *avium-intracellulare* (Left Panel) and the same extract following treatment with n-octanesulphonylacetamide (Right Panel).
Figures 3A and 3B show photomicrographs of *M. bovis* BGC with (B) or without (A) prior n-octanesulphonylacetamide treatment.

### DETAILED DESCRIPTION OF THE EMBODIMENTS

The compounds synthesized according to this invention are based on theoretical transition-state intermediates of the β-ketoacyl synthase reaction of fatty acid synthase (E.C. 2.3.1.85). The β-ketoacyl synthase reaction is common to fatty acid synthesis in prokaryotes and eukaryotes, including *Mycobacteria.* The family of compounds shown in Table 1 was found to be cytotoxic against a variety of mycobacteria, including drug resistant strains. Instead of inhibiting Type I/Type II fatty acid synthesis in mycobacteria, however, Compound HIII-50 (III-50) inhibits the synthesis of mycolic acids, specialized fatty acids found particularly in *Mycobacteria* sp. One of the key steps in mycolic acid synthesis is fatty acid elongation, which employs carbon-carbon condensation similar to the β-ketoacyl synthase reaction in *de novo* fatty acid synthesis. It is believed that these compounds act at this fatty acid elongation step critical to mycolic acid synthesis.

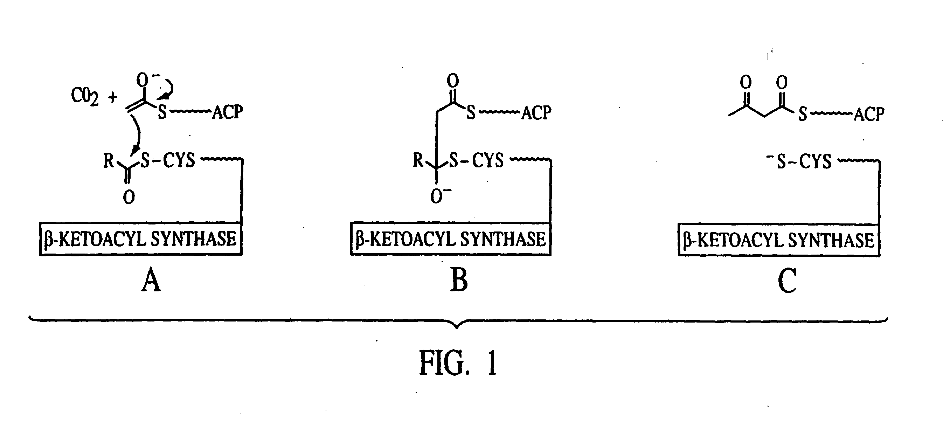

The transition states for all of the two-carbon elongation reactions are presumed, on existing biochemical evidence and mechanistic grounds, to be quite similar (see Figure 1) and involve acylanion formation by decarboxylation of malonyl-CoA/ACP to provide a nucelophile (Figure 1, step A) to react with the thioester-bound acyl chain to generate tetrahedral intermediate (Figure 1, step B). This transient intermediate is anticipated to collapse (Figure 1, step C) to extend the growing acyl unit (RCO-) by two carbons. Further steps then reduce the β-keto group of the ACP/CoA derivative and finally return it to step A to begin the chain extension cycle again.

Low molecular weight organic compounds which are potential mimics of the tetrahedral intermediate (see step B) may be expected to be inhibitors of this biochemical process, and such compounds are represented by Formula I.

R-SOₙ-Z-CO-Y, Formula I

wherein R, n, Z and Y are as defined above.

In preferred compounds according to Formula I, R may be an alkyl group having 6-20 carbons, preferably 8, 10 or 12 carbons, and may be saturated or unsaturated, branched or unbranched, or the alkyl chain may be interrupted by an aromatic ring to give ortho-, meta-, or para-disubstitution. Z is preferably -CH₂-, -CH₂-CH₂-, cis or trans -CH=CH-, -NH-CH₂-, .-CH₂-NH-, -O-CH₂-, or -CH₂-O-, and Y is selected from -NH₂, -O-CH₂-C₆H₅, and -O-CH₃. Between R and Z is a sulfur atom in the form of a sulfoxide or sulfone. The sulfone III-50 and close structural analogues with shorter and longer saturated and unsaturated alkyl side chains, as well as alternative mimics of the tetrahedral intermediate of step B are exemplified but not exclusively represented by the structures shown in Table 1.

The compounds of Formula I may be synthesized by a variety of routes including alkylation of a mercaptoester to produce a thioether (see, e.g., R. L. Smith et al., J. Med. Chem., 1977, 20:540-547), followed by oxidation to afford a sulfoxide or sulfone and conversion of the ester to an amide with ammonia or a substituted amine. A suitable synthetic scheme is shown in Scheme 1 and exemplified in Example 1.

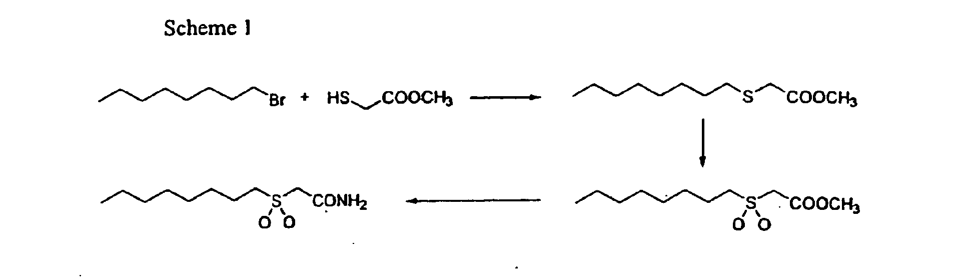

Alternatively, a sulfonate salt, for example the sodium salt, may be reacted with a haloester to give a sulfone (i.e. R-SO₂-Na⁺ + X-(CH₂)ₙ-COOR ---> R-SO₂-(CH₂)ₙ-COOR'; see, e.g., E. Gipstein, C. G. Willson and H. S. Sachdev, J. Org. Chem., 1980, 45:1486-1489), which can be ammonolyzed to the corresponding amide as above. Typically, X=Cl,Br or I. A second alternative would be reaction of a haloamide (X-(CH₂)ₙ-CONH₂ with either a sulfonate salt, as above, or with a thiolate anion followed by oxidation to similarly yield the sulfonyl amide or the sulfoxide amide (see, e.g., S. Huenig and O. Boes, Liebigs Annalen der Chemie, 1953, 579:23-26). It will be apparent to the skilled worker that the sense of these reactions can be reversed so that a halohydrocarbon may be reacted with a mercaptoamide or the salt of a sulfenylamide or sulfenylester to obain the same products. A third alternative would be reaction of a thiol (R-SH) with propiolate ester or amide to form a sulfanyl-acrylic ester or amide followed by oxidation to the sulfoxide or sulfone. Finally, a halo- or mercaptonitriles can be reacted by the above schemes to give thioethers or sulfonylnitriles, which can be hydrolyzed to the amides. Where other alternative synthetic routes to produce the compounds of Formula I occur to the skilled worker, the products of such synthesis are also within the contemplation of this invention.

- The compounds according to Formula I may be used as antibiotics against microbes having in their cell walls α-substituted, β-hydroxy fatty acids, such as corynemycolic acids(e.g., C30), nocardic acids (e.g., C50) or mycolic acids (e.g., C90). Unless otherwise indicated, use of the term mycolic acids herein refers to any of these long chain α-substituted, β-hydroxy fatty acids. In particular, compounds according to Formula I exhibit antibiotic activity against corynebacteria, nocardiae, rhodococcus and mycobacteria. More particularly, these compounds are effective against *Mycobacterium tuberculosis,* drug-resistant *M. tuberculosis, M. avium intracellulare, M. leprae,* or *M. paratuberculosis.*

Preferred compounds according to Formula I will have substantial antibiotic activity against susceptible organisms (see, e.g., Table 1). Antibiotic effectiveness of compounds according to Formula I may be determined as described below or by use of assays described in U.S. Patent No. 5,614,551,

In particular, U.S. Patent 5,614,551 describes an *in vitro* therapeutic index based on comparison of the concentration which inhibits growth of normal fibroblasts to the minimal inhibitory concentration for a compound, and preferred compounds will have an *in vitro* therapeutic index of at least 2, more preferably at least 5, and most preferably at least 10.

Novel drug therapy, using compounds of this invention which are effective against multiply drug resistant tuberculosis, will aid in treating both patients presently suffering from active disease, and the millions of potential patients who harbor quiescent disease which may become active as a result of immunosuppression or other systemic disease. These drugs will also be useful against the "atypical mycobacteria" such as *M. avium-intracellulare,* a common AIDS pathogen, and other species that are commonly drug resistant. Given the biochemical similarity between *M. tuberculosis* and *M. leprae,* these drugs may be expected to be useful in the treatment of leprosy (Hansen's disease). Potential use in livestock or other veterinary applications include treatment of infections by *Mycobacterium paratuberculosis,* also known as Johne's bacillus, an organism that produces a chronic enteritis in ruminants (e.g., cattle and sheep) which is invariably fatal, and *Rhodococcus,* another organism which produces mycolic acids as well as potentially fatal respiratory infections in horses and immunocompromised patients. Treatment of human patients infected with *M. paratuberculosis* is also within the contemplation of this invention.

Treatment according to this invention involves administering the compound of Formula I to the subject of treatment. Pharmaceutical compositions containing any of the compounds of this invention may be administered by parenteral (subcutaneously, intramuscularly, intravenously, intraperitoneally, intrapleurally, intravesicularly or intrathecally), topical, oral, rectal, or nasal or inhalation route, as necessitated by choice of drug, pharmaceutical carrier, and disease.

Therapeutic compounds according to this invention are preferably formulated in pharmaceutical compositions containing the compound and a pharmaceutically acceptable carrier. The concentrations of the active agent in pharmaceutically acceptable carriers will depend on solubilities. The dose used in a particular formulation or application will be determined by the requirements of the particular type of disease and the constraints imposed by the characteristics and capacities of the carrier materials. The pharmaceutical composition may contain other components so long as the other components do not reduce the effectiveness of the compound according to this invention so much that the therapy is negated. Pharmaceutically acceptable carriers are well known, and one skilled in the pharmaceutical art can easily select carriers suitable for particular routes of administration (see, e.g., Remington's Pharmaceutical Sciences, Mack Publishing Co., Easton, PA, 1985).

Dose and duration of therapy will depend on a variety of factors, including the therapeutic index of the drugs, disease type, patient age, patient weight, and tolerance of toxicity. Dose will generally be chosen to achieve serum concentrations from about 1 ng to about 100 µg/ml, typically 0.1 µg/ml to 10 µg/ml. Preferably, initial dose levels will be selected based on their ability to achieve ambient concentrations shown to be effective in *in vitro* models and *in vivo* models and in clinical trials, up to maximum tolerated levels. The dose of a particular drug and duration of therapy for a particular patient can be determined by the skilled clinician using standard pharmacological approaches in view of the above factors. The response to treatment may be monitored by analysis of blood or body fluid levels of the compound according to this invention, measurement of activity of the compound or its levels in relevant tissues or monitoring disease state in the patient. The skilled clinician will adjust the dose and duration of therapy based on the response to treatment revealed by these measurements.

Typically, the compositions described above will be combined or used together or in coordination with one or more other therapeutic substances, e.g., other drugs presently used in treating tuberculosis. The compound of Formula I, or a synergistic combination of inhibitors, will of course be administered at a level (based on dose and duration of therapy) below the level that would kill the animal being treated. Preferably, administration will be at a level that will not irreversibly injure vital organs, or will not lead to a permanent reduction in liver function, kidney function, cardiopulmonary function, gastrointestinal function, genitourinary function, integumentary function, musculoskeletal function, or neurologic function. On the other hand, administration of inhibitors at a level that kills some cells which will subsequently be regenerated (e.g., endometrial cells) is not necessarily excluded.

### EXAMPLES

In order to facilitate a more complete understanding of the invention, a number of Examples are provided below for purposes of illustration only.

### Example 1. Synthesis of substituted sulfonylamides

To illustrate the synthetic method shown in Scheme 1, a 25 g synthesis of III-50 was carried out.
**Methyl n-Octylthioacetate.** Octyl bromide (50.21 g., 0.26 mole), methyl thioglycoate (22.35 ml, 26.53 g, 0.25 mole) and potassium carbonate (34.5 g, 0.25 mole) were charged into a I L round-bottomed flask. To this mixture was added 350 ml of acetone and the suspension was refluxed for 48 h. After cooling to room temperature, the reaction mixture was filtered with the aid of acetone. The filtrate was evaporated under reduced pressure and the residue thus obtained was purified by distillation under reduced pressure. The fraction distilling at 118-121 °C/3.8 mm Hg was collected. Yield: 48 g; 88%
**IR** (**neat**): 2924, 1736, 1435, 1278, 1133, 1011 cm¹.
**¹H NMR** (**CDCl₃**): δ 3.64 (s, 3H), 3.13 (s, 2H), 2.52 (t, 2H, *J=* 7.2 Hz), 1.47 (m, 2H), 1.2-1.4 (m, 10H), 0.88 (t, 3H, *J*= 6.8 Hz).
**¹³C**, **NMR**: **(CDCl₃):** δ 13.9, 22.5, 28.6, 29.0, 29.02, 31.6, 32.5, 33.3, 52.1, 171.0.
**Methyl n-Octanesulphonylacetate.** A 3 L three-necked round-bottomed flask (fitted with a mechanical stirrer) was charged sequentially with ammonium heptamolybdate tetrahydrate (56 g, 0.045 mole), methyl n-octylthioacetate (40 g, 0.183 mole) and 1.5 L absolute alcohol (Schultz, HS, Freyermuth, HB., and Buc, SR, *J. Org. Chem.* **28**:1140-1142, 1963). The vigorously stirred solution was cooled to 0 °C and to this cooled solution was added 104 ml of 30% hydrogen peroxide solution (0.732 mole) over a period of 1 h. The reaction mixture was allowed to warm to room temperature over a period of 2 h and then stirred for another 24 h when thin layer chromatography on silica showed complete disappearance of the starting material. The reaction mixture was filtered and the filtrate was evaporated under reduced pressure. The residue thus obtained was dissolved in ethyl acetate (1L) and washed with water (100 ml x 2), brine (100 ml). The organic layer was dried over anhydrous MgSO₄ and, after filtration of the solvents, was evaporated under reduced pressure to obtain the sulfone as a waxy solid, 36 g, 78%. The produce appeared to be pure (>95%) by NMR and was submitted to the next reaction without further purification.
**IR (neat):** 2919,2848,1743,1460,1437,1329,1278,1218,1137, 1108, 1010, 912, 723 cm⁻¹.
**¹H NMR (CDCl₃):** δ 3.96 (s, 2H), 3.79 (s, 3H), 3.21 (t, 3H, *J*= 8Hz), 1.80 (m, 2H), 1.20-1.45 (m, 10H), 0.81 (t, 3H, *J*= 6.7 Hz).
**¹³C NMR (CDCl₃):** δ 13.9, 21.7, 22.4, 28.2, 28.7, 28.8, 31.5, 53.1, 53.4, 57.0, 163.4.
**n-Octanesulphonylacetamide.** A solution of methyl n-octanesulphonylacetate (35 g, 0.143 mole) in 350 ml of anhydrous methanol was stirred magnetically at room temperature. To this solution was added 24 ml of aqueous ammonium hydroxide (27%, 6.48 g, 0.185 mole) in drops over a period of 30 minutes. The solution was stirred for 24 h and the white precipitate formed was filtered. The solid was recrystallized from hot ethyl acetate to obtain the required acetamide III-50 as a crystalline solid, mp. 140-142 °C, 33g, 97.6%.
**IR (neat):** 3386, 2920, 1659, 1420,1315,1286, 1129, cm⁻¹.
**¹H NMR (CDCl₃):** δ 6.56 (br s, 1H, NH), 5.69 (br s, 1H, NH) 3.86 (s, 2H), 3.14 (app 1:1:1 triplet, J app = 8.0 Hz, 2H), 2.16 (m, 2H), 1.86 (m, 2H), 1.1-1.3 (m, 12H), 0.86 (t, J = 7.1 Hz, 3H).
**¹³C NMR (CD₃COCD₃):** δ 14.7, 22.9, 23.6, 29.4, 30.1, 30.15, 30.8, 32.8, 54.0, 164.8
HRMS calculated for C₁₀H₂₅N₂O₃S (M+NH₄⁻) 253.1586, found 253.1587.

### Example 2. Synthesis of substituted sulfonylamides

To illustrate the synthetic methods shown in Scheme 2 below, the following syntheses of compounds I-31 and 1-89 were carried out.

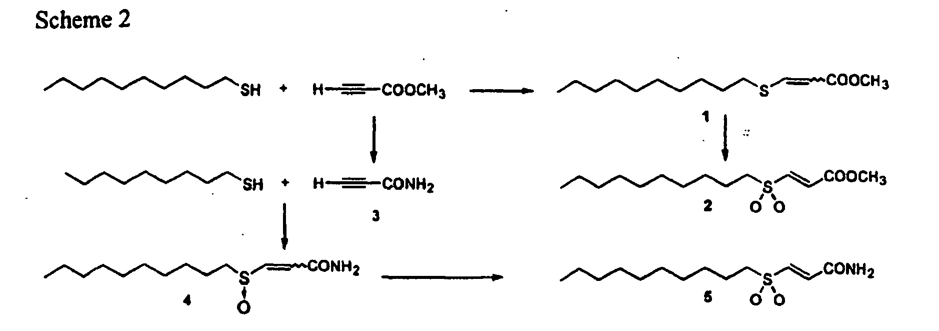

**(*E*/*Z*)-3-decylsulfanyl-acrylic acid methyl ester** (**1**): Triethylamine (0.5 mL, 3.6 mmol) was added dropwise to a solution of decanethiol (2.5 mL, 12.1 mmol) and methyl propiolate (4.3 mL, 48.3 mmol) in dichloromethane (20 mL). The solution was stirred at room temperature for 20 min under argon, after which it was diluted with water (60 mL) and extracted with dichloromethane (3 x 60 mL). The organic layer was washed with water (2 x 60 mL), brine (60 mL), dried over anhydrous magnesium sulfate, and concentrated *in vacuo.* Purification by flash chromatography (silica gel, ethyl acetate:hexanes 1:49) yielded a clear oil (3.06 g, 98%) as a mixture of isomers (*E*:*Z*, 6:1), which was carried through to the next step.
*E isomer:* IR (neat, cm⁻¹): 2920, 2848, 1712, 1580, 1460, 1430, 1300, 1255, 1215, 1160, 1041, 1015, 945, 827, 715, 697.
¹H NMR (CDCl₃): δ 0.88 (*t,* 3H, *J* = 6.7 Hz), 1.26 (*bs,* 12H), 1.40 (*m,* 2H), 1.67 (*quint,* 2H, *J* = 7.3 Hz), 2.78 (*t,* 2H, *J* = 7.4 Hz), 3.72 (*s,* 3H), 5.74 (*d,* 1H, *J* = 15.2 Hz), 7.70 (*d,* 1H, *J=* 15.2 Hz).
*Z isomer*: IR (neat, cm⁻¹): 2920, 2848, 1712, 1580, 1460, 1430, 1300, 1255, 1215, 1160, 1041, 1015, 945, 827, 715, 697.
¹H NMR (CDCl₃) δ 0.88 (*t,* 3H, *J* = 6.7 Hz), 0.9-1.9 (*app. s & several m,* 16H), 2.77 (*t,* 2H, *J* = 7.4 Hz), 3.75 (*s,* 3H), 5.85 (*d,* 1H, *J* = 10.2 Hz), 7.10 (*d,* 1H, *J* = 10.2 Hz).

**(*E*)-3-decylsulfanyl-acrylic acid methyl ester (2):** Compound **1** (0.50 g, 1.93 mmol), was dissolved in methanol (8.0 mL), and cooled to 0°C, added to a solution of oxone (1.84 g, 6.0 mmol) in water (8.0 mL, 49.5%) at 0°C, and stirred for 4h at room temperature. The reaction was then diluted with water (60 mL), and extracted with chloroform (3 x 60 mL). The organic extract was washed with water (2 x 60 mL), brine (60 mL), dried over anhydrous magnesium sulfate, and concentrated *in vacuo.* Purification by flash chromatography (silica gel, ethyl acetate:hexanes 1:1) yielded white crystals (0.53g, 93%) as a mixture of isomers (*E*:*Z*, 6:1). Purification by preparative TLC (ethyl acetate:hexanes 1:1) yielded compound **2** as white crystals.
mp 62-63 °C; IR (CH₂Cl₂, cm⁻¹): 3060, 2950, 2920, 2845, 1730, 1470, 1435, 1280, 1230, 1165,1130, 995, 965, 815, 765, 690, 573.
¹H NMR (CDCl₃) δ 0.88 (*t,* 3H, *J* = 6.7 Hz), 1.27 (*bs,* 12H)*,* 1.43 (*m,* 2H), 1.80 (*sym. m,* 2H), 3.05 (*sym. m,* 2H), 3.86 (*s,* 3H), 6.88 (*d,* 1H, *J* = 15.3 Hz), 7.34 (*d,* 1H, *J* = 15.3 Hz). Calculated for C₁₄H₂₆O₄S: C, 57.90, H, 9.02, S, 11.04. Found: C, 57.94, H, 9.12, S, 11.16.

**Propiolamide (3):** Methyl propiolate (2.0 mL, 22.5 mmol) was added to liquid ammonia at -78°C and stirred for 2 hours. Evaporation at room temperature yielded compound **3** as white crystals (1.46 g, 94%).
mp 58-61°C (lit.[5], 61-62°C); ¹H NMR (D₂O) δ 3.50 (*s*, 1H), 4.43 (*bs,* 2H).

**(*E*/*Z*)-3-decylsulfanyl-propionamide (4):** Triethylamine (0.5 mL, 3.6 mmol) was added dropwise to a solution of decanethiol (1.36 mL, 6.56 mmol) and propiolamide (1.80 g, 26.1 mmol) in dichloromethane (20 mL). The solution was stirred at room temperature for 20 min under argon, after which it was diluted with water (60 mL) and extracted with dichloromethane (3 x 60 mL). The organic layer was washed with water (2 x 60 mL), brine (60 mL), dried over anhydrous magnesium sulfate, and concentrated *in vacuo.* Purification by flash chromatography (silica gel, ethyl acetate:hexanes 3:1) yielded compound **4** as white crystals (1.45g, 91%) as a mixture of isomers (*E:Z,* 1:10), which was carried through to the next step.
*Mixture of isomers:* mp 65-70°C; IR (CH₂Cl₂, cm⁻¹): 3400, 3330, 3280, 3200, 2920, 2840, 1725, 1650, 1570, 1460, 1400, 1300, 1190, 770.
*E-isomer:* ¹H NMR (CDCl₃) δ 0.89 (*t,* 3H, *J*= 6.8 Hz), 1.27 (*bs*, 12H), 1.41 (*m*, 2H), 1.70 (*m,* 2H), 2.74 (*t,* 2H, *J*= 7.5 Hz). 5.4 (*bs,* 2H, NH₂), 5.82 (*d*,1H*, J*=10.1 Hz), 6.95 (*d,* 1H, *J* = 10.1 Hz).

**(*E*)-3-decylsulfonyl-propionamide (5):** Compound **4** (0.50 g, 2.05 mmol) was dissolved in methanol (8.0 mL), cooled to 0°C and added to a solution of oxone (1.84 g, 6.0 mmol) in water (8.0 mL, 49.5%) at 0°C and stirred for 4 h at room temperature. The solution was then diluted with water (60 mL), and extracted with chloroform (3 x 60 mL). The organic extract was washed with water (2 x 60 mL), brine (60 mL), dried over magnesium sulfate, and concentrated *in vacuo.* Purification by flash chromatography (silica gel, ethyl acetate:hexanes 17:3) yielded compound **5** as white crystals (0.52 g, 92%) as a mixture of isomers (*E:Z*, 6:1). Purification by preparative TLC (ethyl acetate:hexanes 3:1) yielded compound **5** as white crystals.
mp 148-149 °C; IR (CHCl₃, cm⁻¹): 3390, 3140, 3060, 2920, 2840, 1700, 1615, 1455, 1395, 1320, 1130, 960, 814.
¹H NMR (CDCl₃): δ 0.089 (*t,* 3H, *J* = 6.8 Hz), 1.27 (*bs*, 12H),1.43 (*m*, 2H), 1.80 (*m,* 2H) 3.05 (*sym. m*, 2H), 5.67 *(bs,* 1H, NH), 5.85 (*bs,* 1H, NH), 6.94 (*d,* 1H, *J* = 14.8 Hz), 7.35 (*d,* 1H, *J*=14.8 Hz).
Calculated for C₁₃H₂₅NO₃S: C, 56.70, H. 9.15, N 5.09, S 11.64. Found: C, 56.78, H, 9.16, N, 5.04, S, 11.76.

### Example 3. In vitro activity of sulfones and sulfoxide against mycobacteria

Various compounds were tested as described in U.S. Patent No. 5,614,551 to determine minimum inhibitory concentratios (MIC) of the compounds against drug-resistant MTB (*M. tuberculosis* strain H37Rv), *M. avium-intracellular,* and *M. bovis* BCG. The results are shown in Table 1. Dose-response curves for the compound, designated III-50, have demonstrated an MIC of 6.5 µg/ml against the virulent strain of *M. tuberculosis,* H37Rv, and 6.25 µg/ml for *M. bovis* BCG. Dose-response curves for the compound designated S-I-73 have demonstrated an MIC of 3.12 µg/ml against MTB and 12.50 µg/ml against *M. avium-intracellular.*

### Example 4. III-50 inhibits mycolic acid synthesis via a target different from isoniazid.

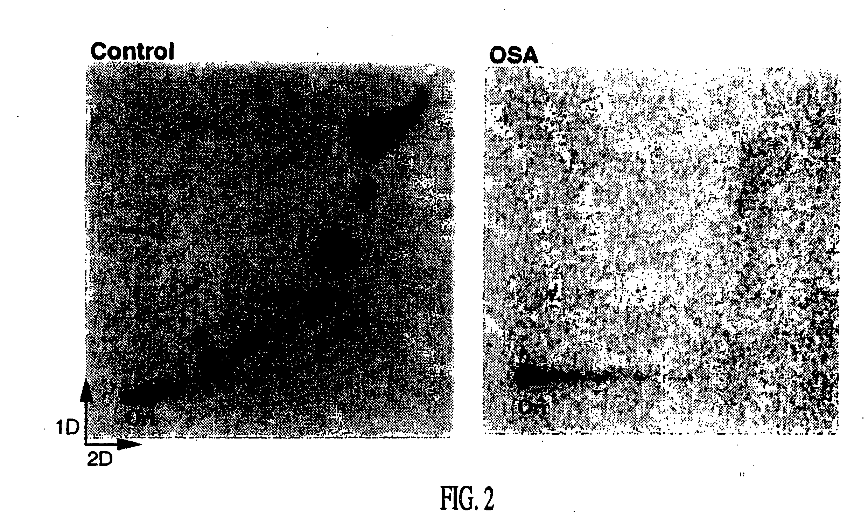

In a series of metabolic labeling experiments, the activity of a number of lipid metabolic pathways were studied in the presence and absence of III-50 using two-dimensional thin-layer chromatography and phosphorimage quantification. The TLC plates were spotted with mycobacterial acid methanolysates and developed in the first direction with petroleum ether (bp 60-80°C):acetone (95:5, v/v, 3 times) and in the second direction with toluene:acetone (97:3), v/v, once). Abbreviations in Figure 2 are: Ori = origin; A = α-mycolate; B = ketomycolate; and C = ω-mycolate. The left hand panel shows acid methanolysates from *M. avium-intracellulare* control cultures. The right hand panel shows acid methanolysates from *M. avium-intracel*/*u*/*are* cultures treated with 12.5 µg/ml n-octanesulphonylacetamide (III-50).

While minor qualitative and quantitative alterations of various lipid species were identified, the most profound effect was noted upon mycolic acid synthesis. Reproductions of the phosphorimages (Figure 2) demonstrate that in the presence of III-50 mycolic acids are undetectable in *M*. *avium-intracellulare* and significantly reduced if *M. bovis* BCG. More importantly, while inhibition of mycolic acid synthesis is thought to be the mechanism of action of isoniazid (Takayama, et al., 1972 & 1975; Quemard, et al., 1993 & 1996; and Baldock, et al.), III-50 inhibits both the growth of *M. avium-intracellulure,* which is routinely isoniazid resistant (>2.5 µg/ml), and also an isoniazid (INH) resistant *M. tuberculosis* (>0.4 µg/ml). Thus, although both isoniazid and III-50 inhibit mycolic acid synthesis, the enzymatic target of III-50 within the mycolic acid synthetic pathway appears to differ from that of isoniazid.

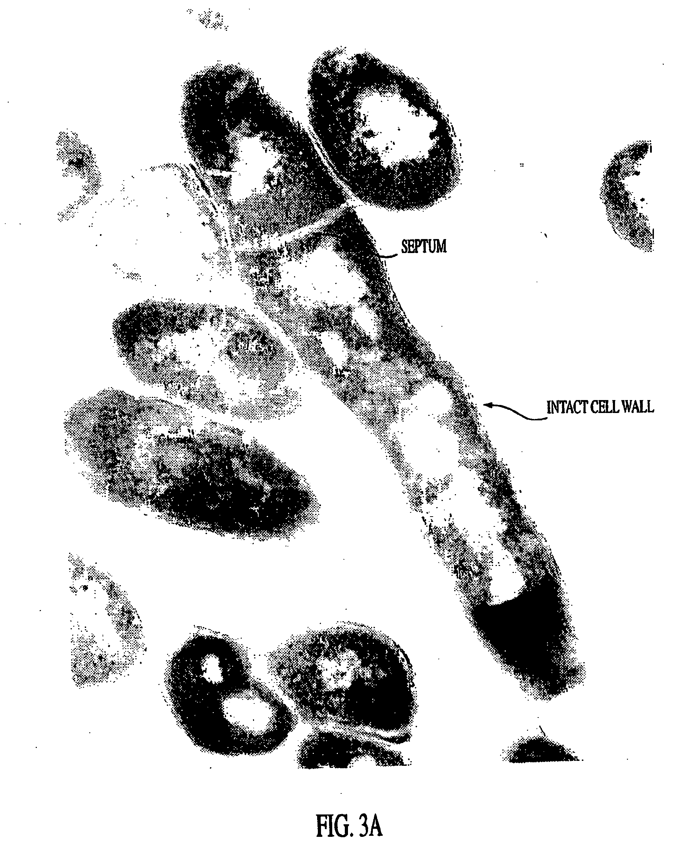

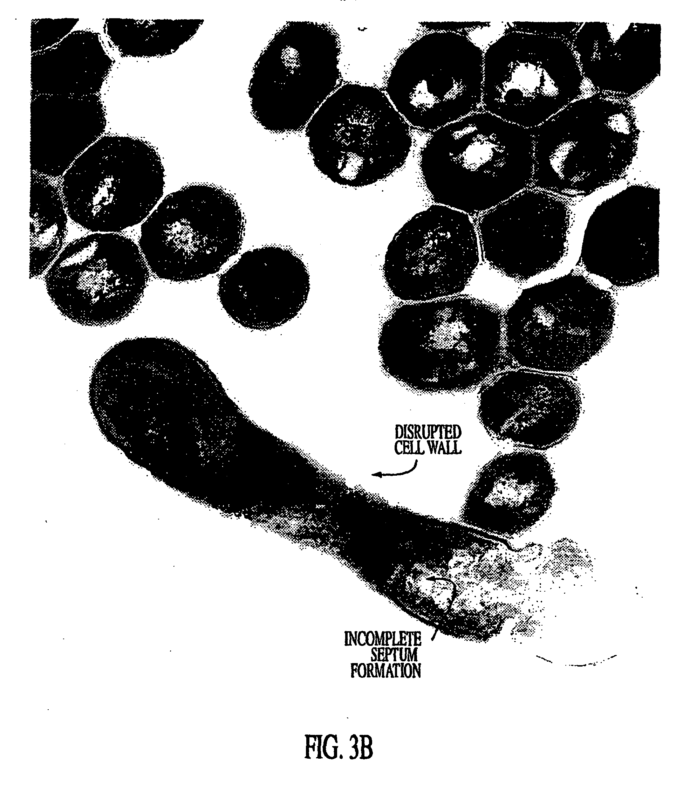

Inhibition of mycolic acid synthesis leads to the disruption of the cell wall in mycobacteria. Figure 3 shows electron micrographs which depict cell division of *M. bovis* BCG in the presence (Panel A) and absence (Panel B) of III-50. Note, in the control, the well-developed cell wall and septum as the bacterium divides. In contrast, in the presence of III-50, there is disruption of the cell wall likely as a result of inhibition of mycolic acid synthesis.

**Table 1. In Vitro Activities of Sulfones and Sulfoxides against M. tuberculosis**

| | | MIC (ug/ml) | | |
|---|---|---|---|---|
| Compound # | | MTB | BCG | MAI |
| SI-73 | 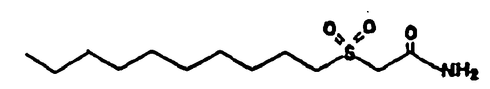 | 3.12 | | 12.50 |
| HIII-50 | 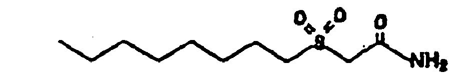 | 6.25 | 6.25 | 25.00 |
| SI-46 | 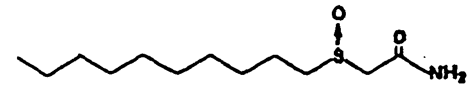 | 6.25 | | |
| SI-52 | 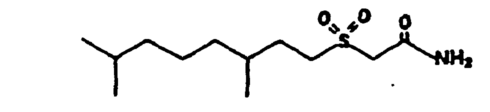 | 6.25 | | |
| HIII-206a | 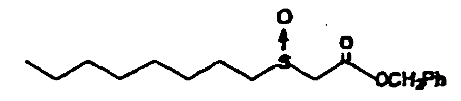 | 6.25 | | |
| HIII-206b | 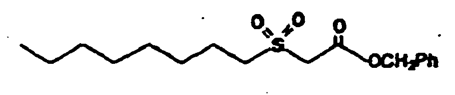 | 12.50 | | |
| DI-59 | 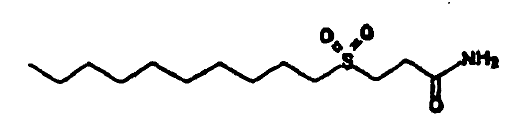 | >25 | | |
| JRG-I-89-2 | 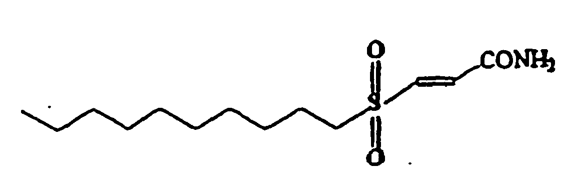 | 12.5 | | |
| JRG-I-89-1 | 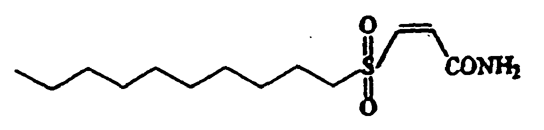 | 25 | | |
| JRG-I-89-3 (mixture of cis and trans) | 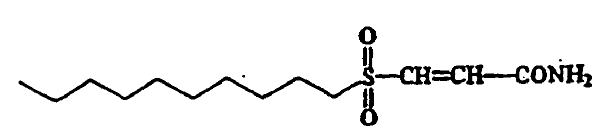 | 6.25 | | |

| | | | | |
|---|---|---|---|---|
| MTB = *M*. *tuberculosis* (strain H37Rv) BCG = *M.bovis* MAI *= M.avium intracellulare* See legend in Appendix for Methods. | | | | |

## Claims

1. A compound having a formula:
R-SOₙ-Z-CO-Y,
wherein:
R is an alkyl group having 6-20 carbon atoms or an alkyl group having 6-20 carbon atoms interrupted by an aromatic ring;
Z is a radical selected from the group consisting of -CH₂-, two of these radicals coupled together, and -CH=CH-;
Y is selected from -NH₂, and O-CH₂-C₆H₅; and
n is 1 or 2; and wherein the compound is not CH₃(CH₂)₁₁SO₂CH₂CONH₂.

2. A compound according to claim 1, wherein R is an alkyl group having 6 to 20 carbon atoms and said alkyl group is a branched alkyl group.

3. A compound according to claim 1 or claim 2, wherein R is an alkyl group having 8, 10, or 12 carbon atoms.

4. A use of a compound having the formula:
R-SOₙ-Z-CO-Y
wherein:
R is selected from the group consisting of unsaturated hydrocarbon groups having 6-20 carbon atoms and alkyl groups having 6-20 carbon atoms interrupted by an aromatic ring;
Z is a radical selected from the group consisting of -CH₂-, -CH₂CH₂-, -NH-NH-, -O-, -NH-,-O-NH-. -CH₂-NH-, -CH₂O-, -NH-O-, -NH-CH₂-, -O-CH₂-, and -CH=CH-;
Y is selected from the group consisting of-NH_{2,} -O-CH₂-C₆H₅ and -O-CH₃; and
n is 1 or 2;
in the preparation of a medicament for inhibiting the growth of a microbial cell wherein the cell synthesises α-substituted β-hydroxy fatty acids.

5. Use according to claim 4, wherein R is selected from alkyl groups having 6-20 carbon atoms interrupted by an aromatic ring to give ortho-, meta-, or para-disubstitution.

6. Use according to claim 4 or claim 5, wherein the microbial cell is selected from the group consisting of corynebacteria, nocardiae, rhodococcus, and mycobacteria.

7. Use according to any of claims 4 to 6, wherein R is a branched alkyl group.

8. Use according to any of claims 4 to 6, wherein R is an n-alkyl group.

9. Use according to any of claims 4 to 8, wherein n is 1.

10. Use according to any of claims 4 to 9, wherein Z is -CH₂-.

11. Use according to any of claims 4 to 10, wherein Y is -NH₂.

12. Use according to any of claims 4 to 11, wherein: R is -(CH₂)₉-CH₃ or -(CH₂)₇-CH₃, n is 2, Z is -CH₂-, and Y is -NH₂.

13. Use according to any of claims 4 to 11, wherein R is selected from the group consisting of alkyl groups having 6-10 carbon atoms, unsaturated hydrocarbon groups having 6-10 carbon atoms, or alkyl groups having 6-10 carbon atoms interrupted by at least one aromatic ring.

14. Use according to any of claims 4 to 13, wherein the microbial cell is selected from the group consisting *of Mycobacteria tuberculosis,* drug resistant *M. tuberculosis, M. avium intracellulare, M. leprae,* and *M. Paratuberculosis.*

15. Use according to any of claims 4 to 14, wherein the microbial cell is pathogenic *Mycobacteria sp.*

16. Use according to any of claims 4 to 15, wherein the medicament is administered to an animal.

17. Use according to claim 16, wherein the animal is selected from the group consisting of humans, ruminants and horses.

18. Use according to claim 17, wherein the ruminant is selected from the group consisting of sheep and cattle.

19. A compound having a formula:
R-SOₙ-Z-CO-Y,
wherein:
R is an alkyl group having 6-20 carbon atoms or an alkyl group having 6-20 carbon atoms interrupted by an aromatic ring;
Z is a radical selected from the group consisting of -CH₂-, two of these radicals coupled together, and -CH=CH-;
Y is selected from -NH₂, and O-CH₂-C₆H₅;
and n is 1 or 2;
for use as a medicament.

## Patentansprüche

1. Verbindung der Formel:
R-SOₙ-Z-CO-Y,
wobei:
R eine Alkylgruppe mit 6-20 Kohlenstoffatomen oder eine durch einen aromatischen Ring unterbrochene Alkylgruppe mit 6-20 Kohlenstoffatomen ist;
Z ein Rest ist, der ausgewählt ist aus der Gruppe bestehend aus -CH₂-, zwei dieser Reste miteinander gekoppelt und -CH=CH-;
Y ausgewählt ist aus -NH₂ und -O-CH₂-C₆H₅; und
n 1 oder 2 ist; und
wobei es sich bei der Verbindung nicht um CH₃(CH₂)₁₁SO₂CH₂CONH₂ handelt.

2. Verbindung nach Anspruch 1, wobei R eine Alkylgruppe mit 6 bis 20 Kohlenstoffatomen ist und die Alkylgruppe eine verzweigte Alkylgruppe ist.

3. Verbindung nach Anspruch 1 oder 2, wobei R eine Alkylgruppe mit 8, 10 oder 12 Kohlenstoffatomen ist.

4. Verwendung einer Verbindung der Formel:
R-SOₙ-Z-CO-Y,
wobei:
R aus der Gruppe ausgewählt ist, die aus ungesättigten Kohlenwasserstoffgruppen mit 6-20 Kohlenstoffatomen und durch einen aromatischen Ring unterbrochenen Alkylgruppen mit 6-20 Kohlenstoffatomen besteht;
Z ein Rest ist, der ausgewählt ist aus der Gruppe bestehend aus -CH₂-, -CH₂CH₂-, -NH-NH-, -O-, -NH-, -O-NH-, -CH₂-NH-, -CH₂O-, -NH-O-, -NH-CH₂-, -O-CH₂- und -CH=CH-;
Y ausgewählt ist aus der Gruppe bestehend aus -NH₂, -OCH₂-C₆H₅ und -O-CH₃; und
n 1 oder 2 ist,
zur Herstellung eines Medikaments zur Inhibierung des Wachstums einer mikrobiellen Zelle, wobei die Zelle α-substituierte β-Hydroxyfettsäuren synthetisiert.

5. Verwendung nach Anspruch 4, wobei R aus Alkylgruppen mit 6-20 Kohlenstoffatomen ausgewählt ist, die so durch einen aromatischen Ring unterbrochen sind, dass sich eine ortho-, meta- oder para-Disubstitution ergibt.

6. Verwendung nach Anspruch 4 oder 5, wobei die mikrobielle Zelle ausgewählt ist aus der Gruppe bestehend aus Corynebakterien, Nocardien, Rhodococcus und Mycobakterien.

7. Verwendung nach einem der Ansprüche 4 bis 6, wobei R eine verzweigte Alkylgruppe ist.

8. Verwendung nach einem der Ansprüche 4 bis 6, wobei R eine n-Alkylgruppe ist.

9. Verwendung nach einem der Ansprüche 4 bis 8, wobei n = 1 ist.

10. Verwendung nach einem der Ansprüche 4 bis 9, wobei Z =-CH₂- ist.

11. Verwendung nach einem der Ansprüche 4 bis 10, wobei Y = -NH₂ ist.

12. Verwendung nach einem der Ansprüche 4 bis 11, wobei R = -(CH₂)₉-CH₃ oder -(CH₂)₇-CH₃ ist, n = 2 ist, Z = -CH₂- ist und Y = -NH₂ ist.

13. Verwendung nach einem der Ansprüche 4 bis 11, wobei R aus der Gruppe ausgewählt ist, die aus Alkylgruppen mit 6-10 Kohlenstoffatomen, ungesättigten Kohlenwasserstoffgruppen mit 6-10 Kohlenstoffatomen und durch wenigstens einen aromatischen Ring unterbrochenen Alkylgruppen mit 6-10 Kohlenstoffatomen besteht.

14. Verwendung nach einem der Ansprüche 4 bis 13, wobei die mikrobielle Zelle aus der Gruppe ausgewählt ist, die aus *Mycobacterium tuberculosis,* medikamentenresistentem *Mycobacterium tuberculosis, M. avium intracellulare, M. leprae* und *M. paratuberculosis* besteht.

15. Verwendung nach einem der Ansprüche 4 bis 14, wobei es sich bei der mikrobiellen Zelle um pathogenes *Mycobacterium* sp. handelt.

16. Verwendung nach einem der Ansprüche 4 bis 15, wobei das Medikament einem Tier verabreicht wird.

17. Verwendung nach Anspruch 16, wobei das Tier aus der Gruppe ausgewählt ist, die aus Menschen, Wiederkäuern und Pferden besteht.

18. Verwendung nach Anspruch 17, wobei der Wiederkäuer aus der Gruppe ausgewählt ist, die aus Schafen und Rindern besteht.

19. Verbindung der Formel:
R-SOₙ-Z-CO-Y,
wobei:
R eine Alkylgruppe mit 6-20 Kohlenstoffatomen oder eine durch einen aromatischen Ring unterbrochene Alkylgruppe mit 6-20 Kohlenstoffatomen ist;
Z ein Rest ist, der ausgewählt ist aus der Gruppe bestehend aus -CH₂-, zwei dieser Reste miteinander gekoppelt und -CH=CH-;
Y ausgewählt ist aus -NH₂ und -O-CH₂-C₆H₅; und
n 1 oder 2 ist;
zur Verwendung als Medikament.

## Revendications

1. Composé répondant à la formule :
R-SOₙ-Z-CO-Y,
dans laquelle :
R représente un groupe alkyle ayant 6 à 20 atomes de carbone ou un groupe alkyle ayant 6 à 20 atomes de carbone interrompu par un noyau aromatique ;
Z représente un radical choisi dans le groupe consistant en un radical -CH₂-, deux de ces radicaux couplés l'un avec l'autre, et un radical -CH=CH- ;
Y est choisi entre des groupes -NH₂ et O-CH₂-C₆H₅ ; et
n est égal à 1 ou 2 ; le composé n'étant pas le composé de formule CH₃(CH₂)₁₁SO₂CH₂CONH₂.

2. Composé suivant la revendication 1, dans lequel R représente un groupe alkyle ayant 6 à 20 atomes de carbone et ledit groupe alkyle est un groupe alkyle ramifié.

3. Composé suivant la revendication 1 ou la revendication 2, dans lequel R représente un groupe alkyle ayant 8, 10 ou 12 atomes de carbone.

4. Utilisation d'un composé répondant à la formule :
R-SOₙ-Z-CO-Y,
dans laquelle :
R est choisi dans le groupe consistant en des groupes hydrocarbonés insaturés ayant 6 à 20 atomes de carbone et un groupe alkyle ayant 6 à 20 atomes de carbone interrompu par un noyau aromatique ;
Z représente un radical choisi dans le groupe consistant en des radicaux -CH₂-, -CH₂CH₂-, -NH-NH-, -O-, -NH-, -O-NH-, -CH₂-NH, -CH₂O- -NH-O-, -NH-CH₂-, -O-CH₂ et -CH=CH- ;
Y est choisi entre des groupes -NH₂, O-CH₂-C₆H₅ et -O-CH₃ ; et
n est égal à 1 ou 2 ;
dans la préparation d'un médicament destiné à inhiber la croissance d'une cellule microbienne, ladite cellule synthétisant des β-hydroxy-acides gras α-substitués.

5. Utilisation suivant la revendication 4, dans laquelle R est choisi parmi des groupes alkyle ayant 6 à 20 atomes de carbone interrompus par un noyau aromatique pour obtenir une disubstitution en position ortho, méta ou para.

6. Utilisation suivant la revendication 4 ou la revendication 5, dans laquelle la cellule microbienne est choisie dans le groupe consistant en des Corynébactéries, des Nocardia, des Rhodococcus et des Mycobactéries.

7. Utilisation suivant l'une quelconque des revendications 4 à 6, dans laquelle R représente un groupe alkyle ramifié.

8. Utilisation suivant l'une quelconque des revendications 4 à 6, dans laquelle R représente un groupe n-alkyle.

9. Utilisation suivant l'une quelconque des revendications 4 à 8, dans laquelle n est égal à 1.

10. Utilisation suivant l'une quelconque des revendications 4 à 9, dans laquelle Z représente un groupe -CH₂-.

11. Utilisation suivant l'une quelconque des revendications 4 à 10, dans laquelle Y représente un groupe -NH₂.

12. Utilisation suivant l'une quelconque des revendications 4 à 11, dans laquelle : R représente un groupe - (CH₂)₉-CH₃ ou -(CH₂)₇-CH₃, n est égal à 2, Z représente un groupe -CH₂- et Y représente un groupe -NH₂.

13. Utilisation suivant l'une quelconque des revendications 4 à 11, dans laquelle R est choisi dans le groupe consistant en des groupes alkyle ayant 6 à 10 atomes de carbone, des groupes hydrocarbonés insaturés ayant 6 à 10 atomes de carbone et des groupes alkyle ayant 6 à 10 atomes de carbone interrompus par au moins un noyau aromatique.

14. Utilisation suivant l'une quelconque des revendications 4 à 13, dans laquelle la cellule microbienne est choisie dans le groupe consistant en *Mycobacterium tuberculosis, M. tuberculosis* résistant aux médicaments, *M. avium intracellulare, M. leprae* et *M. paratuberculosis.*

15. Utilisation suivant l'une quelconque des revendications 4 à 14, dans laquelle la cellule microbienne est un *Mycobacterium sp.* pathogène.

16. Utilisation suivant l'une quelconque des revendications 4 à 15, dans laquelle le médicament est administré à un animal.

17. Utilisation suivant la revendication 16, dans laquelle l'animal est choisi dans le groupe consistant en les êtres humains, les ruminants et les chevaux.

18. Utilisation suivant la revendication 17, dans laquelle le ruminant est choisi dans le groupe consistant en les moutons et les bovins.

19. Composé répondant à la formule :
R-SOₙ-Z-CO-Y,
dans laquelle :
R représente un groupe alkyle ayant 6 à 20 atomes de carbone ou un groupe alkyle ayant 6 à 20 atomes de carbone interrompu par un noyau aromatique ;
Z représente un radical choisi dans le groupe consistant en un radical -CH₂-, deux de ces radicaux couplés l'un avec l'autre, et un radical -CH=CH- ;
Y est choisi entre des groupes -NH₂ et O-CH₂-C₆H₅ ; et
n est égal à 1 ou 2 ;
destiné à être utilisé comme médicament.
